# EUROPEAN PATENT APPLICATION

(11) **EP 4 692 036 A1**
(43) Date of publication of application: **11.02.2026**
(21) Application number: 24850248.6
(22) Date of filing: 25.06.2024
(51) Int. Cl.: C07C 29/78, C07C 29/04, C07C 31/10

(54) **METHOD FOR PREPARING ISOPROPYL ALCOHOL**

(30) Priority: 21.09.2023 KR 20230126458
(71) Applicant: LG Chem, Ltd., Seoul 07336 (KR)
(72) Inventor: JANG, Kyung Soo, 34122 Daejeon (KR); LEE, Sung Kyu, 34122 Daejeon (KR); HWANG, Sung June, 34122 Daejeon (KR)
(74) Representative: Goddar, Heinz J.
(86) International application number: PCT/KR2024/008762
(87) International publication number: WO 2025/063438

(57) **Abstract**

The present invention relates to a method of preparing isopropyl alcohol, the method including: supplying a feed stream including a propylene monomer and water to a reaction unit and allowing a reaction to proceed to produce a reaction product including isopropyl alcohol, a propylene monomer, and water; supplying each of a first stream including a vapor phase reaction product and a second stream including a liquid phase reaction product that are discharged from the reaction unit to an absorption column of a purification unit; and discharging a third vapor phase stream including the propylene monomer through an upper portion of the absorption column to be circulated to the reaction unit, and discharging a fourth liquid phase stream including water and isopropyl alcohol through a lower portion of the absorption column to be fed to an isopropyl alcohol purification unit, wherein the purification unit includes one or more heat exchangers, and when the third stream is circulated to the reaction unit, the third stream is converted from a vapor phase to a liquid phase through the one or more heat exchangers provided in the purification unit, and then transferred.

## Description

### [Technical Field]

### CROSS-REFERENCE TO RELATED APPLICATION

The present application claims the benefit of and priority to Korean Patent Application No. 10-2023-0126458, filed on September 21, 2023, the entire contents of which are incorporated herein by reference.

### Technical Field

The present invention relates to a method of preparing isopropyl alcohol, and more particularly, to a method of introducing a liquefaction process when recovering an unreacted product in separating isopropyl alcohol from a reaction product of an isopropyl alcohol preparation process and effectively recovering an unreacted product, thereby reducing the amount of energy used.

### [Background Art]

Isopropyl alcohol (IPA) is used for various purposes including a solvent for a cleaning agent, a raw material for an industrial paint or reagent, a paint, an ink, or the like in the electronics industry such as the manufacture of a semiconductor, a liquid crystal display (LCD), or the like.

In the process of preparing isopropyl alcohol, for example, propylene and water are used as raw material components. In this case, the propylene and water react to produce isopropyl alcohol. A reaction product in the isopropyl alcohol preparation process includes isopropyl alcohol, an unreacted propylene monomer, and unreacted water. In this case, the isopropyl alcohol is separated and recovered from the reaction product in the isopropyl alcohol preparation process, and the unreacted propylene monomer is recovered and reused in the isopropyl alcohol preparation process.

In this respect, an absorption column is generally used to separate the isopropyl alcohol and the unreacted propylene monomer from the reaction product in the isopropyl alcohol preparation process. Specifically, the isopropyl alcohol preparation process is performed through a vapor phase reaction. At this time, a vapor phase reaction product to be produced is fed through a lower portion of the absorption column, isopropyl alcohol in the reaction product is dissolved using water as a solvent and separated through the lower portion of the absorption column, and a vapor phase stream including a propylene monomer is separated through an upper portion of the absorption column and recycled to a reactor. In this case, in order to recycle the vapor phase stream including the propylene monomer to the reactor, the vapor phase stream needs to be compressed to a high pressure through an additional device such as a compressor, and the use of a pipe having a large diameter is essential because the vapor phase stream has a large volumetric flow rate. However, the high-pressure compression process for transferring the vapor phase stream is difficult to operate and maintain, and consumes a lot of energy due to a high amount of electricity used, which increases facility maintenance costs and energy costs.

Therefore, there is a need for a method for securing economic efficiency and achieving energy savings by improving the efficiency of the process of recycling the absorption column upper discharge vapor phase stream including the unreacted propylene monomer to the reactor in the isopropyl alcohol preparation process.

### [Disclosure]

### [Technical Problem]

In order to solve the problems mentioned in the background art, an object of the present invention is to provide a method for securing economic efficiency and achieving energy savings by improving circulation efficiency in a process of recycling an absorption column upper discharge vapor phase stream including an unreacted propylene monomer to a reactor during an isopropyl alcohol preparation process.

However, the problems to be solved by the present application are not limited to the object described above, and other problems not described will be clearly understood by those skilled in the art from the following description.

### [Technical Solution]

In one general aspect, provided is a method of preparing isopropyl alcohol that includes: supplying a feed stream including a propylene monomer and water to a reaction unit and allowing a reaction to proceed to produce a reaction product including isopropyl alcohol, a propylene monomer, and water; supplying each of a first stream including a vapor phase reaction product and a second stream including a liquid phase reaction product that are discharged from the reaction unit to an absorption column of a purification unit; and circulating a third vapor phase stream including the propylene monomer discharged through an upper portion of the absorption column to the reaction unit, and supplying a fourth liquid phase stream including water and isopropyl alcohol discharged through a lower portion of the absorption column to an isopropyl alcohol purification unit.

In addition, in the method of preparing isopropyl alcohol according to the present invention, the purification unit may include one or more heat exchangers, and when the third stream is circulated to the reaction unit, the third stream may be converted from a vapor phase to a liquid phase through the one or more heat exchangers provided in the purification unit, and then transferred.

### [Advantageous Effects]

According to the method of preparing isopropyl alcohol of the present invention, the reaction product in the isopropyl alcohol preparation process is fed to the absorption column to separate an unreacted propylene monomer and isopropyl alcohol, and the vapor phase stream including the unreacted propylene monomer discharged through the upper portion of the absorption column is liquefied and circulated to the reaction unit, such that a volumetric flow rate of the stream may be effectively reduced, thereby improving the circulation efficiency of the unreacted monomer in the process. Furthermore, a plurality of heat exchangers are provided downstream of the absorption column to induce heat exchange between the streams, such that the amount of refrigerant used may be reduced, and heat recovery may also be maximized.

The effects that may be obtained by the present application are not limited to the effects described above, and other effects not described above may be clearly understood by those skilled in the art to which the present invention pertains from the following description.

### [Description of the Drawings]

FIG. 1 is a process flow diagram of a method of preparing isopropyl alcohol according to an embodiment of the present invention.
FIG. 2 is a process flow diagram of a method of preparing isopropyl alcohol according to an embodiment of the present invention.
FIG. 3 is a process flow diagram of a method of preparing isopropyl alcohol according to a comparative example.

### [Detailed Description]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

With regard to the description of the drawings, similar reference numerals may be used to refer to similar or related components.

A singular form of a noun corresponding to an item may include one or more of the items, unless the relevant context clearly indicates otherwise.

In the present disclosure, each of such phrases as "A or B", "at least one of A and B", "at least one of A or B", "A, B, or C", "at least one of A, B, and C", and "at least one of A, B, or C" may include any one of, or all possible combinations of the items enumerated together in one of the corresponding phrases.

The term "and/or" includes a combination of a plurality of related components or any one of the plurality of related components.

In addition, the terms such as "1st" and "2nd" or "first" and "second" may be used to simply distinguish a corresponding component from another component, and does not limit the corresponding components in other aspects (for example, importance or order).

In addition, the terms such as "front surface", "rear surface", "upper surface", "lower surface", "side surface", "left side", "right side", "upper portion", and "lower portion" used in the present application are defined based on the drawings, and the shape and position of each component are not limited by the terms.

The terms "include" or "have" specify the presence of features, numerals, steps, operations, components, parts described in the present disclosure, or a combination thereof, but do not preclude the presence or addition of one or more other features, numerals, steps, operations, components, parts, or a combination thereof.

When a component is said to be "connected", "coupled", "supported", or "contacted" with another component, this includes not only when components are directly connected, coupled, supported, or contacted, but also when components are indirectly connected, coupled, supported, or contacted through a third component.

When a component is "on" another component, this includes not only when the component is in contact with another component, but also when there is another component between two components.

In addition, the terms "about" and "substantially" used in the present application are used in a numerical value or in the vicinity of the numerical value in the meanings mentioned when inherent manufacturing and material allowable errors are presented, and are used to prevent unconscious infringers from illegally using the accurate or absolute numbers disclosed in the present invention to help understanding of the present invention.

The term "stream" used in the present application may refer to a flow of a fluid in a process, and may also refer to a fluid flowing through a pipe itself. Specifically, the stream may refer to both a fluid flowing through a pipe connecting respective devices to each other itself and a flow of a fluid. In addition, the fluid may include any one or more components of gas, liquid, and solid.

The term "upper portion" used in the present application refers to a point at a height of 0% to 20% from the uppermost portion of a device to a lower portion, and specifically, may refer to the top (of the column), unless otherwise specified. In addition, the term "lower portion" refers to a point at a height of 80% to 100% from the uppermost portion of the device to the lower portion, and specifically, may refer to the bottom (of the column).

In addition, the "pressure" referred to in the present application refers to gauge (g) pressure measured based on atmospheric pressure.

An embodiment of the present invention relates to a method of preparing isopropyl alcohol (IPA). Hereinafter, the method of preparing isopropyl alcohol of the present invention will be described in detail with reference to the drawings.

According to the present invention, a method of preparing isopropyl alcohol is provided. The method of preparing isopropyl alcohol of the present invention includes: producing a reaction product in a reaction unit 100, supplying the reaction product to an absorption column 210; and recovering an unreacted monomer in the reaction product from the absorption column 210 to the reaction unit 100.

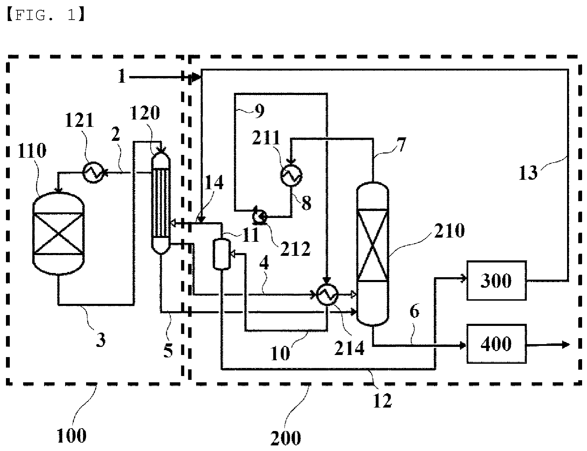

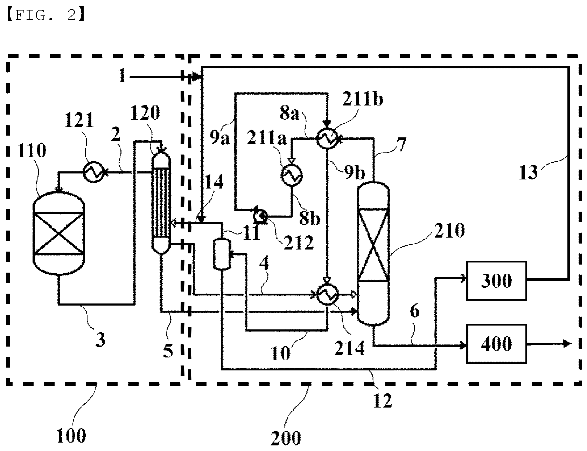

FIGS. 1 and 2 are each a process flow diagram of a method of preparing isopropyl alcohol according to an embodiment of the present invention.

The isopropyl alcohol may be produced through a vapor phase reaction between a propylene monomer and water. Specifically, a feed stream 1 including a propylene monomer and water may be fed to the reaction unit 100, and the reaction product produced in the reaction unit 100 may include isopropyl alcohol, an unreacted propylene monomer, and unreacted water. In this case, the isopropyl alcohol is separated and recovered from the reaction product, and the unreacted propylene monomer is recovered and reused in the isopropyl alcohol preparation process.

In the method of preparing isopropyl alcohol according to the present invention, first, the feed stream 1 including the propylene monomer and water is fed to the reaction unit 100 and a reaction is allowed to proceed to produce a reaction product including isopropyl alcohol. The reaction product may further include an unreacted propylene monomer, water, and a side reaction product.

According to an embodiment of the present invention, a molar ratio (water/propylene monomer) of the water and the propylene monomer fed to the reaction unit 100 may be 0.3 to 0.5, specifically 0.35 to 0.5, and more specifically 0.35 to 0.45. When the molar ratio in the stream fed to the reaction unit 100 is low, a conversion rate to isopropyl alcohol may increase, but this may increase a by-product production rate. That is, when the molar ratio increases, the conversion rate to isopropyl alcohol may decrease, but a high selectivity may be obtained.

According to an embodiment of the present invention, the feed stream 1 including the propylene monomer and water is fed to a reactor 110 of the reaction unit 100, and a vapor phase reaction is allowed to proceed in the reactor 110, such that a propylene monomer and water, which are unreacted products, isopropyl alcohol, which is a reaction product, and a side reaction product may be obtained. Isopropyl alcohol is produced through the vapor phase reaction in the reactor 110, such that the amount of by-products produced by a side reaction may be reduced.

According to an embodiment of the present invention, the reactor 110 may be operated under optimal conditions in which isopropyl alcohol may be efficiently prepared through the vapor phase reaction between the propylene monomer and water. For example, an operating pressure of the reactor 110 may be about 30 kg/cm²·G to 50 kg/cm²·G, preferably 35 kg/cm²·G to 50 kg/cm²·G, and more preferably 35 kg/cm²·G to 45 kg/cm²·G, and an operating temperature of the reactor 110 may be about 180°C to 220°C, preferably 185°C to 220°C, and more preferably 190°C to 215°C. The reactor is operated at the pressure and temperature in the above ranges, such that isopropyl alcohol may be effectively produced through the vapor phase reaction using the propylene monomer and water.

Meanwhile, the feed stream 1 including the propylene monomer and water may be further heated through a heating means 121 and then fed to the reactor 110. The heating means 121 may be located upstream of the reactor 110 and may be used as a heat source for the isopropyl alcohol production reaction. More specifically, a temperature of the feed stream may be adjusted to a reactor introduction temperature condition through the heating means and then may be fed to the reactor. For example, the heating means 121 may be a heater that transfers heat to a feed stream 2 by passing a high temperature/high pressure vapor (steam), hot water, a heat transfer fluid, or the like through a jacket, and there is no particular limitation on the heating means.

The propylene monomer and water are subjected to the vapor phase reaction in the reactor 110, such that a vapor phase reaction product 3 may be discharged from the reactor 110. In this case, a temperature of the vapor phase reaction product 3 discharged from the reactor 110 may be, for example, 200°C to 220°C, 205°C to 220°C, or 205°C to 215°C.

The reaction unit 100 may further include one or more heat exchangers 120. Referring to FIG. 1, the feed stream 1 may be fed to the reactor 110 after passing through the heat exchanger 120 provided in the reaction unit 100.

According to an embodiment of the present invention, a part of the vapor phase reaction product discharged from the reactor 110 may be condensed into a liquid phase reaction product while passing through one or more heat exchangers 120, and the remainder may be present as a vapor phase reaction product. As an example, the vapor phase reaction product discharged from the reactor 110 may pass through the heat exchanger 120 of the reaction unit and may be separated into a first stream 4 including a vapor phase reaction product and a second stream 5 including a liquid phase reaction product. In this case, the first stream 4 and the second stream 5 may be separated and discharged through a separate pipe formed in the heat exchanger 120 of the reaction unit, or may be separated through a vapor-liquid separation device installed downstream of the heat exchanger 120 of the reaction unit.

According to an embodiment of the present invention, the vapor phase reaction product 3 discharged from the reactor 110 may be heat-exchanged with a feed stream 14 fed to the reactor 110 in one or more heat exchangers 120. Here, the feed stream 14 that is heat-exchanged with the reaction product 3 in the heat exchanger 120 acts as a cooling medium, and more specifically, may be a mixed stream 14 further including recycle streams 11 and 13 as unreacted raw materials, which will be described below, in addition to the feed stream 1 including a fresh raw material. Specifically, a part of the vapor phase reaction product 3 discharged from the reactor 110 may be condensed while passing through one or more heat exchangers 120, and the feed stream 14 may be heated while passing through one or more heat exchangers 120 before being fed to the reactor 110. In this case, a temperature of the feed stream 1, before passing through the one or more exchangers 120, may be, for example, 90°C to 130°C, specifically 110°C to 120°C, and more specifically 105°C to 115°C. In addition, a temperature of the feed stream 2, after passing through the one or more exchangers 120, may be, for example, 160°C to 180°C, and specifically 165°C to 175°C. Meanwhile, a temperature of each of the first stream 4 and the second stream 5 obtained by passing the vapor phase reaction product 3 discharged from the reactor 110 through one or more heat exchangers 120 may be, for example, 110°C to 130°C, and specifically 115°C to 125°C.

More specifically, a heat exchanger having a large shell diameter and a large number of tubes may be used as the heat exchanger 120 of the reaction unit. For example, the shell diameter of the heat exchanger of the reaction unit may be 700 mm to 900 mm, and preferably 750 mm to 850 mm, and the number of tubes of the heat exchanger of the reaction unit may be 600 to 800, and preferably 650 to 750. By satisfying the shell diameter and the number of tubes, an internal cross-sectional area of the heat exchanger 120 is increased to improve a heat transfer area. Accordingly, a heat recovery performance for the vapor phase reaction product 3 discharged from the reactor may be maximized, such that a heat exchange rate may be increased.

As such, the vapor phase reaction product stream 3 discharged from the reactor 110 is heat-exchanged with the mixed stream 14 of the recycle streams 11 and 13 and the feed stream 1 introduced into the reaction unit 100, which will be described below, such that the vapor phase reaction product stream 3 may be separated into the first stream 4 including a vapor phase reaction product, and the second stream 5 including a liquid phase reaction product that are discharged from the reactor 110, and at the same time, the feed stream may be preheated and fed to the reactor 110. Here, the first recycle stream 11 introduced into the reaction unit may be a vapor phase stream, the second recycle stream 13 and the feed stream 1 introduced into the reaction unit may be a liquid phase stream, and the mixed stream 14 in which these streams are mixed may be a vapor-liquid mixed stream. Through this, energy for supplying the feed stream to the reactor 110 and heating the feed stream may be saved, and temperatures and compositions of the first stream 4 and the second stream 5 may be controlled, such that separation efficiency in a subsequent separation process using the absorption column may be increased.

According to an embodiment of the present invention, the first stream 4 may include 85 wt% to 95 wt% of a propylene monomer, 4 wt% to 8 wt% of isopropyl alcohol, and 1 wt% to 5 wt% of water. Specifically, in the first stream 4, an amount of the propylene monomer is significantly high, and amounts of the isopropyl alcohol and water are significantly low.

In addition, the second stream 5 may include 0.5 wt% to 5 wt% of a propylene monomer, 5 wt% to 15 wt% of isopropyl alcohol, and 80 wt% to 90 wt% of water. Specifically, in the second stream 5, an amount of the propylene monomer is significantly low, and an amount of the water is significantly high. In this case, an amount of the isopropyl alcohol included in the second stream 5 may be higher than the amount of the isopropyl alcohol included in the first stream 4.

According to an embodiment of the present invention, a ratio of a flow rate of the first stream 4 to a flow rate of the second stream 5 discharged from the reaction unit 100 may be 1:5 to 1:15, 1:7 to 1:12, or 1:8 to 1:10. By cooling the vapor phase reaction product stream discharged from the reactor 110 to a temperature of 110°C to 130°C, the ratio of the flow rate of the first stream 4 to the flow rate of the second stream 5 may be controlled to 1:5 to 1:15. Here, the "flow rate" may refer to a flow of a weight per unit hour. As a specific example, a unit of the flow rate may be ton/hr.

The first stream 4 including a vapor phase reaction product and the second stream 5 including a liquid phase reaction product that are discharged from the reaction unit 100 are each fed to the absorption column 210 of a purification unit 200. More specifically, as described above, the vapor phase reaction product stream 3 discharged from the reactor 110 may pass through one or more heat exchangers 120 provided in the reaction unit and may be separated into the first stream 4 including a vapor phase reaction product and the second stream 5 including a liquid phase reaction product, and the first stream 4 and the second stream 5 may each be fed to a lower side of the absorption column 210.

In addition, the purification unit 200 may include a heat exchanger 214 in the upstream process of the absorption column 210. When the first stream 4 is fed to the absorption column 210, the first stream 4 including a vapor phase reaction product may be partially condensed through the first heat exchanger 214 provided in the purification unit 200 and fed to the absorption column 210 as a vapor-liquid mixed phase. For example, in the heat exchanger 214, the first stream 4 may be condensed by using a separate refrigerant or by exchanging heat with a stream in the process. For example, the first stream 4 may be cooled in the first heat exchanger 214 by heat exchange with streams 9 and 9b obtained by liquefying an upper discharge stream of the absorption column, which will be described below, and converting the upper discharge stream to a liquid phase or a vapor-liquid mixed phase.

According to an embodiment of the present invention, the total number of stages of the absorption column 210 may be 10 to 30, specifically 15 to 30, and more specifically 15 to 25. For example, when the total number of stages of the absorption column 210 is 10, the first stream 4 and the second stream 5 may be fed to the 10th stage of the absorption column 210.

According to an embodiment of the present invention, the reaction product fed from the reaction unit 100 is fed to the absorption column 210, and a lower discharge stream 6 including isopropyl alcohol, and an upper discharge stream 7 including an unreacted propylene monomer, may be separated in the absorption column 210. More specifically, the reaction product may be fed through a lower portion of the absorption column 210, isopropyl alcohol in the reaction product may be dissolved using water as a solvent to be separated through the lower portion of the absorption column 210, and a stream including an unreacted propylene monomer may be separated through an upper portion of the absorption column 210.

The solvent used in the absorption column 210 may be, for example, water. By using water, which is a component used in the reaction, as the solvent, a separate device for separating the solvent in the downstream process is not required.

A flow rate of the solvent fed to the absorption column 210 may be 20% to 60%, specifically 25% to 55%, and more specifically 30% to 50%, of the total flow rate of the reaction product fed to the absorption column 210. The solvent is fed at a flow rate within the above range, such that the absorption capacity of isopropyl alcohol included in the reaction product fed to the absorption column 210 may be improved, and at the same time, the energy costs for solvent recovery in the downstream process may be prevented from excessively increasing.

According to an embodiment of the present invention, an operating pressure of the absorption column 210 may be 20 kg/cm²·G to 40 kg/cm² ·G, specifically 25 kg/cm²·G to 40 kg/cm² ·G, and more specifically 25 kg/cm²·G to 35 kg/cm²·G, and an operating temperature of the absorption column 210 may be 80°C to 110°C, specifically 90°C to 110°C, and more specifically 90°C to 100°C. The absorption column 210 is operated at the pressure and temperature in the above ranges, such that the upper discharge stream including an unreacted propylene monomer and the lower discharge stream including isopropyl alcohol may be effectively separated.

According to an embodiment of the present invention, the third vapor phase stream 7 including a propylene monomer discharged through the upper portion of the absorption column 210 may be circulated to the reaction unit 100. In this case, a temperature of the third vapor phase stream 7 may be 85°C to 105°C, and specifically 90°C to 100°C.

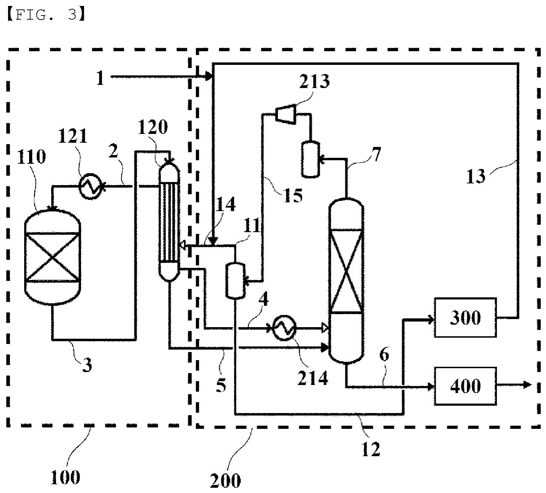

FIG. 3 is a process flow diagram of a method of preparing isopropyl alcohol according to a comparative example. The method of preparing isopropyl alcohol is performed through a vapor phase reaction. In this case, a vapor phase reaction product to be produced is fed through the lower portion of the absorption column 210, isopropyl alcohol in the reaction product is dissolved using water as a solvent and separated through the lower discharge stream 6 of the absorption column, and a vapor phase stream including a propylene monomer is separated through the upper discharge stream 7 of the absorption column and recycled to the reactor 110. In this case, in general, in order to recycle the vapor phase upper discharge stream 7 of the absorption column including a propylene monomer to the reactor 110, since a volumetric flow rate of the vapor phase stream is large, as illustrated in FIG. 3, the vapor phase stream needs to be compressed to a high pressure through a compressor 213. However, even when the vapor phase stream is compressed to a high pressure, there is a limit to reducing the volumetric flow rate of the vapor phase stream, and thus, the use of a pipe having a large internal diameter is essential. In addition, the high-pressure compression process for transferring the vapor phase stream is difficult to operate and maintain, and consumes a lot of energy due to a high amount of electricity used, which increases facility maintenance costs and energy costs.

In the present invention, in order to solve the above problems, in the isopropyl alcohol preparation process, in particular, the stream 7 discharged as a vapor phase through the upper portion of the absorption column 210 may be liquefied through one or more heat exchangers 211, 211a, and 211b and transferred, such that the volumetric flow rate may be effectively reduced. Through this, the circulation efficiency of the unreacted monomer in the process may be improved.

According to an embodiment of the present invention, the purification unit 200 is provided with one or more heat exchangers 211, 211a, and 211b downstream of the third stream 7 discharged through the upper portion of the absorption column 210. Accordingly, when the third stream 7 is circulated to the reaction unit 100, the third stream 7 may be converted from a vapor phase to a liquid phase through one or more heat exchangers 211a and 211b provided in the purification unit 200, and the converted third stream 7 may be transferred. For example, in the heat exchangers 211, 211a, and 211b provided in the purification unit, the third vapor phase stream 7 may be liquefied by using a separate refrigerant or by heat exchange with a stream in the process. In addition, the third stream 7 is liquefied, such that the unreacted propylene monomer may be circulated to the reaction unit 100 using a pump 212, and a pipe size may be reduced due to a reduction in volumetric flow rate.

As illustrated in FIG. 1, when one heat exchanger 211 is provided downstream of the absorption column 210, the liquid phase conversion of the third stream 7 may be performed, for example, by condensing, in the heat exchanger 211 provided in the purification unit 200, the third stream 7 discharged through the upper portion of the absorption column 210 using a separate refrigerant. In this case, a third condensed stream 8 may be a liquid phase stream, and a temperature of the third condensed stream 8 may be, for example, 50°C to 75°C, and specifically 60°C to 70°C. In addition, the third liquid phase stream 8 may be compressed through the pump 212, and a compressed liquid phase stream 9 may be fed to the first heat exchanger 214.

As illustrated in FIG. 2, when two heat exchangers 211a and 211b are provided downstream of the absorption column 210, the liquid phase conversion of the third stream 7 may be performed, for example, by primarily condensing the third stream 7 discharged through the upper portion of the absorption column 210 through the second heat exchanger 211b provided in the purification unit 200, and then secondarily condensing the primarily condensed third stream 8a through the third heat exchanger 211a provided in the purification unit 200. In this case, the primarily condensed third stream 8a may be a vapor-liquid mixed phase stream, and the secondarily condensed third stream 8b may be a liquid phase stream. For example, a temperature of the primarily condensed third stream 8a may be 70°C to 90°C, and specifically 75°C to 85°C, and a temperature of the secondarily condensed third stream 8b may be 50°C to 75°C, and specifically 60°C to 70°C. As such, when the third vapor phase stream 7 is liquefied using two heat exchangers 211a and 211b, there is an advantage in that the amount of refrigerant used in the heat exchanger may be reduced and the heat recovery may also be maximized.

Referring to FIG. 2, for example, the third vapor phase stream 7 may be primarily condensed into a vapor-liquid mixed phase by exchanging heat with the secondarily condensed third liquid phase stream 9 through the second heat exchanger 211b, and the primarily condensed third stream 8a in the vapor-liquid mixed phase may be secondarily condensed into a liquid phase in the third heat exchanger 211a using a separate refrigerant. In addition, the stream 8b liquefied by the secondary condensation may be compressed through the pump 212, and a compressed liquid phase stream 9a may be heat-exchanged with the third vapor phase stream 7 in the second heat exchanger 211b and may be converted to the vapor-liquid mixed phase stream 9b again. For example, a temperature of a vapor-liquid mixed phase stream 10 may be 70°C to 95°C, and specifically 80°C to 90°C.

According to an embodiment of the present invention, a volumetric flow rate of each of the liquefied third streams 8 and 8b relative to a volumetric flow rate of the third vapor phase stream 7 may be 10% to 20%, specifically 12% to 18%, and more specifically 13% to 17%. In this case, an internal diameter of a pipe for the liquefied third streams 8 and 8b relative to an internal diameter D of a pipe for the third vapor phase stream 7 may be about (1/4)D to (3/4)D, and specifically (1/3)D to (1/2)D. As such, when the unreacted propylene monomer discharged through the upper portion of the absorption column is circulated to the reaction unit 100, the volumetric flow rate is reduced through liquefaction of the third stream 7, such that the size of the transfer pipe may be significantly reduced.

The liquefied third streams 8 and 8b may be fed to the reaction unit 100 using the pump 212. Previously, as illustrated in FIG. 3, the compressor 213 was used to transfer the vapor phase stream to the reaction unit 100. However, in the present invention, the vapor phase upper stream of the absorption column is liquefied through the heat exchanger, such that the vapor phase upper stream of the absorption column may be transferred to the reaction unit 100 using the pump 212. When the pump 212 is used instead of the compressor, the operation and maintenance become easier and the cost-effectiveness of the device is achieved. In addition, when the upper stream of the absorption column is liquefied and transferred through the pump 212, the amount of electricity used may be reduced to less than about 10% compared to the case of using the compressor 213, and thus, significant energy savings may be achieved.

According to an embodiment of the present invention, the liquefied third streams 9 and 9b may be heat-exchanged with the first stream 4 through the first heat exchanger 214. Referring to FIG. 1, when one heat exchanger 211 is used, the liquid phase stream 9 transferred through the pump 212 may be heat-exchanged with the first vapor phase stream 4 through the first heat exchanger 214 and may be converted to a vapor phase or vapor-liquid phase mixed stream 10. Furthermore, referring to FIG. 2, when two heat exchangers 211a and 211b are used, the secondarily condensed third liquid phase stream 8b may be converted to a vapor-liquid mixed phase in the second heat exchanger 211b again, and the vapor-liquid mixed phase stream 9b may be heat-exchanged with the first stream 4 through the first heat exchanger 214 and may be converted to a vapor phase. A temperature of the vapor phase stream 10, after passing through the first heat exchanger 214, may be, for example, 80°C to 100°C, and specifically 85°C to 95°C. The streams 10 and 11 recycled to the reaction unit 100 need to be fed to the reaction unit after recovering as much heat as possible through the heat exchanger to reduce the amount of heat source (steam) used in the heating means 121, and as a result, the amount of energy used may be reduced.

Furthermore, as illustrated in FIGS. 1 and 2, the third stream 10, after passing through the first heat exchanger 214, may be branched, a part of the stream 11 may be fed to the reaction unit 100, and a remaining stream 12 may be fed to a gas purification unit 300. In this case, both the partial stream 11 and the remaining stream 12 may be vapor phase streams. More specifically, the stream 11 fed to the reaction unit 100 may be mixed with the liquid phase feed stream 1 and then fed to the reaction unit 100 as the vapor-liquid mixed phase stream 14, and a part of the stream 12 fed to the gas purification unit 300 may be recycled to the reaction unit 100 as the purified second recycle stream 13 including an unreacted propylene monomer after going through the gas purification process. That is, the partial vapor phase stream (that is, the first recycle stream) 11 branched from the third stream 10, after passing through the first heat exchanger 214, may be mixed with the second recycle stream 13 purified in the gas purification unit 300 and the liquid phase feed stream 1 and may be fed to the reaction unit 100 as the vapor-liquid mixed phase stream 14.

Meanwhile, according to an embodiment of the present invention, the fourth liquid phase stream 6 including water and isopropyl alcohol discharged through the lower portion of the absorption column 210 may be fed to an isopropyl alcohol purification unit 400.

According to an embodiment of the present invention, the lower discharge stream of the absorption column 210 may include a small amount of an unreacted propylene monomer in addition to isopropyl alcohol and unreacted water. For example, an amount of the unreacted propylene monomer included in the lower discharge stream of the absorption column 210 may be 5 wt% or less, and specifically 3 wt% to 5 wt%. Accordingly, the entire or a part of the lower discharge stream including isopropyl alcohol may be fed to a flash drum and/or a gas purification column from the absorption column 210 to separate the unreacted propylene monomer included in the lower discharge stream of the absorption column 210 as an upper discharge stream, and the lower discharge stream including isopropyl alcohol and unreacted water may be fed to the isopropyl alcohol purification unit 400.

For example, the fourth stream 6 discharged through the lower portion of the absorption column 210 may pass through one or more flash drums before being fed to the isopropyl alcohol purification unit 400. More specifically, as for the fourth stream 6 discharged through the lower portion of the absorption column 210, a part of the fourth stream 6 may be fed to the gas purification unit 300 using a flash drum, and the remaining stream may be fed to the isopropyl alcohol purification unit 400. In addition, the unreacted propylene monomer included in the fourth stream 6 may be separated through an upper discharge stream of the flash drum and fed to the gas purification unit 300, and isopropyl alcohol may be fed to the isopropyl alcohol purification unit 400 through a lower discharge stream of the flash drum.

Furthermore, the upper discharge stream of the flash drum may be purified once more through the gas purification column of the gas purification unit 300, a part of the stream 13 including an unreacted propylene monomer may be discharged through an upper portion of the gas purification column of the gas purification unit 300 and recycled to the reaction unit 100, and the remaining stream including isopropyl alcohol discharged through a lower portion of the gas purification column of the gas purification unit 300 may be fed to the isopropyl alcohol purification unit 400. In this case, it is preferable to vent inert gases such as ethane and propane that should not accumulate in the process.

According to an embodiment of the present invention, in the method of preparing isopropyl alcohol, if necessary, additional devices such as a distillation column, a condenser, a reboiler, a valve, a pump, a separator, and a mixer may be further installed and used.

Hereinabove, the method of preparing isopropyl alcohol according to the present invention has been described and illustrated in the drawings. However, the description and the illustration of the drawings are for only essential components for understating the present invention, and processes and devices not separately described and illustrated may be properly applicable and used for implementing the method of preparing isopropyl alcohol of the present invention, in addition to the processes and devices described and illustrated in the drawings.

Hereinafter, the present invention will be described in more detail with reference to examples. However, the following examples are provided for illustrating the present invention in more detail, and the scope of the present invention is not limited by the following examples.

### [Examples]

### Example 1

As shown in the process flow diagram illustrated in FIG. 1, a process of preparing a reaction product including isopropyl alcohol (IPA) and separating isopropyl alcohol from the reaction product was simulated using Aspen Plus available from Aspen Technology Inc.

Specifically, a feed stream 1 was fed at a flow rate of 5 ton/hr to a reactor 110 operated at a pressure of 40 kg/cm²·G and a temperature of 192°C to 208°C, a molar ratio of water and a propylene monomer fed to a reaction unit 100 was controlled to 0.4, and ethane and propane were included as inert gases.

A vapor phase reaction product stream 3 discharged from the reactor 110 passed through one heat exchanger 120 of the reaction unit and was separated into a first vapor phase stream 4 and a second liquid phase stream 5 and discharged, a part of the first stream 4 was condensed into a liquid phase in a first heat exchanger 214 and then fed through a lower portion of an absorption column 210, and the second stream 5 was fed through the lower portion of the absorption column 210.

The absorption column 210 was operated at a temperature of 90°C to 100°C and a pressure of 31 kg/cm²·G to 32 kg/cm²·G, and isopropyl alcohol in the reaction product stream was absorbed using water fed through an upper portion of the absorption column 210 as a solvent, and thus, the reaction product stream was separated into a third stream 7, which is an upper discharge stream including a propylene monomer, and a fourth stream 6, which is a lower discharge stream including water and isopropyl alcohol.

Specifically, the third vapor phase stream 7 discharged through the upper portion of the absorption column 210 was cooled to a temperature of 60°C to 70°C in a heat exchanger 211 to be liquefied (8), and the liquid phase stream 9 obtained by compressing the third stream 7 liquefied by a pump 212 was heat-exchanged with the first stream 4 in the first heat exchanger 214 and then refluxed to the reaction unit 100. Meanwhile, the fourth stream 6 discharged through the lower portion of the absorption column 210 was fed to an IPA purification unit to obtain isopropyl alcohol from which water was removed.

### Example 2

As shown in the process flow diagram illustrated in FIG. 2, a process of preparing a reaction product including isopropyl alcohol (IPA) and separating isopropyl alcohol from the reaction product was simulated using Aspen Plus available from Aspen Technology Inc.

Specifically, a feed stream 1 was fed at a flow rate of 5 ton/hr to a reactor 110 operated at a pressure of 40 kg/cm²·G and a temperature of 192°C to 208°C, a molar ratio of water and a propylene monomer fed to a reaction unit 100 was controlled to 0.4, and ethane and propane were included as inert gases.

A vapor phase reaction product stream 3 discharged from the reactor 110 passed through a heat exchanger 120 of the reaction unit and was separated into a first vapor phase stream 4 and a second liquid phase stream 5 and discharged, a part of the first stream 4 was condensed into a liquid phase in a first heat exchanger 214 and then fed through a lower portion of an absorption column 210, and the second stream 5 was fed through the lower portion of the absorption column 210.

The absorption column 210 was operated at a temperature of 90°C to 100°C and a pressure of 31 kg/cm² ·G to 32 kg/cm²·G, and isopropyl alcohol in the reaction product stream was absorbed using water fed through an upper portion of the absorption column 210 as a solvent, and thus, the reaction product stream was separated into a third stream 7, which is an upper discharge stream including a propylene monomer, and a fourth stream 6, which is a lower discharge stream including water and isopropyl alcohol.

Specifically, the third vapor phase stream 7 discharged through the upper portion of the absorption column 210 was cooled to a temperature of 60°C to 70°C through second and third heat exchangers 211a and 211b to be liquefied, the liquid phase stream 8b was compressed by a pump 212, a compressed liquid phase stream 9a passed through the second heat exchanger 211b again to exchange heat with the third stream 7, and a stream 9b that passed through the second heat exchanger 211b was heat-exchanged with the first stream 4 in the first heat exchanger 214 and then refluxed to the reaction unit 100. Meanwhile, the fourth stream 6 discharged through the lower portion of the absorption column 210 was fed to an IPA purification unit to obtain isopropyl alcohol from which water was removed.

### Comparative Example 1

As shown in the process flow diagram illustrated in FIG. 3, a process of preparing a reaction product including isopropyl alcohol (IPA) and separating isopropyl alcohol from the reaction product was simulated using Aspen Plus available from Aspen Technology Inc.

Specifically, a feed stream 1 was fed at a flow rate of 5 ton/hr to a reactor 110 operated at a pressure of 40 kg/cm² ·G and a temperature of 192°C to 208°C, a molar ratio of water and a propylene monomer fed to a reaction unit 100 was controlled to 0.4, and ethane and propane were included as inert gases.

A vapor phase reaction product stream 3 discharged from the reactor 110 passed through a heat exchanger 120 of the reaction unit and was separated into a first vapor phase stream 4 and a second liquid phase stream 5 and discharged, a part of the first stream 4 was condensed into a liquid phase in a first heat exchanger 214 and then fed through a lower portion of an absorption column 210, and the second stream 5 was fed through the lower portion of the absorption column 210.

The absorption column 210 was operated at a temperature of 90°C to 100°C and a pressure of 31 kg/cm² ·G to 32 kg/cm²·G, and isopropyl alcohol in the reaction product stream was absorbed using water fed through an upper portion of the absorption column 210 as a solvent, and thus, the reaction product stream was separated into a third stream 7, which is an upper discharge stream including a propylene monomer, and a fourth stream 6, which is a lower discharge stream including water and isopropyl alcohol.

Specifically, the third vapor phase stream 7 discharged through the upper portion of the absorption column 210 was compressed by a compressor 213, and then the compressed vapor phase stream 15 was refluxed to the reaction unit 100. Meanwhile, the fourth stream 6 discharged through the lower portion of the absorption column 210 was fed to an IPA purification unit to obtain isopropyl alcohol from which water was removed.

### Experimental Examples

Table 1 shows a comparison between the phases, temperatures, and relative volumetric flow rates of the streams after passing through the pump 212 or the compressor 213, and the relative amounts of electricity used, which are consumed by the pump 212 or the compressor 213, in the examples and the comparative examples. Here, each of the relative volumetric flow rates and amount of electricity used of Examples 1 and 2 was based on the value of Comparative Example 1 as 100%, and the relative values were shown.

**[Table 1]**

| Classification | | Example 1 (FIG. 1) | Example 2 (FIG. 2) | Comparative Example 1 (FIG. 3) |
|---|---|---|---|---|
| Streams (9, 9a, 15) after absorption column upper compression | Phase | Liquid phase | Liquid phase | Vapor phase |
| | Temperature | 70°C | 70°C | 120°C |
| | Volumetric flow rate | 15.2% | 15.2% | 100% |
| Amount of electricity used by pump 212 or compressor 213 | | 5.1% | 5.1% | 100% |

Referring to Table 1, in the case where the conditions of the vapor phase stream 7 discharged through the upper portion of the absorption column were the same, when the volumetric flow rate of Comparative Example 1 in which the vapor phase stream 7 was transferred to the reaction unit 100 using the compressor 213 was 100%, the relative volumetric flow rates of Examples 1 and 2, in which the vapor phase stream 7 was liquefied through the heat exchangers 211, 211a, and 211b and then transferred to the reaction unit 100 using the pump 212, were significantly reduced to 15.2%. Accordingly, it was confirmed that in Examples 1 and 2, compared to Comparative Example 1, a smaller-sized pipe was able to be used for stream transfer, and thus, the process efficiency and process cost savings were achieved.

In addition, when the amount of electricity used of Comparative Example 1, in which the vapor phase upper discharge stream of the absorption column was compressed by the compressor 213 and then transferred, was 100%, the relative amounts of electricity used of Examples 1 and 2, in which the vapor phase upper discharge stream of the absorption column was converted to a liquid phase and then transferred using the pump 212, were significantly reduced to 5.1%, and thus, it was confirmed that the amount of electricity used was able to be significantly reduced.

In addition, Table 2 shows a comparison of the relative amounts of refrigerant used, which were consumed in the cooling heat exchangers 211 and 211a in which the refrigerant was used to liquefy the vapor phase upper discharge stream 7 of the absorption column, in Examples 1 and 2. Here, the relative amount of refrigerant used was expressed by taking the amount of refrigerant used of Example 1 as 100% and comparing the relative amount of refrigerant used in Example 2 with the amount of refrigerant used of Example 1.

**[Table 2]**

| Classification | Example 1 (FIG. 1) | Example 2 (FIG. 2) |
|---|---|---|
| Amount of refrigerant used | 100% | 79% |
| Cooling and heat recovery heat exchanger | - | Heat exchanger (211b) |

Referring to Table 2, it was confirmed that, in Example 2, in which two cooling heat exchangers 211a and 211b were provided, the amount of refrigerant used was reduced by about 21% compared to Example 1 in which only one cooling heat exchanger 211 was provided to liquefy the vapor phase upper discharge stream of the absorption column. More specifically, in Example 2, in addition to the heat exchanger 211a using a refrigerant, the vapor phase upper discharge stream 7 of the absorption column and the stream 9a that was liquefied through the heat exchanger 211a using the refrigerant and transferred were heat-exchanged with each other through the additional heat exchanger 211b, and therefore, the cooling and heat recovery of the vapor phase upper discharge stream 7 of the absorption column were achieved simultaneously, thereby reducing a rate of use of the refrigerant.

Although the exemplary embodiments of the present invention have been described, the present invention is not limited to these exemplary embodiments, and those skilled in the art will appreciate that various modifications and alterations may be made without departing from the concept and scope of the claims described below.

### [Detailed Description of Main Elements]

| | | | |
|---|---|---|---|
| 100: | Reaction unit | 200: | Purification unit |
| 300: | Gas purification unit | 400: | Isopropyl alcohol purification unit |
| 110: | Reactor | 210: | Absorption column |
| 120: | Heat exchanger of reaction unit | 121: | Heating means |
| 211, 211a, 211b, 214: | Heat exchanger of purification unit | | |
| 212: | Pump | 213: | Compressor |

## Claims

1. A method of preparing isopropyl alcohol, the method comprising:
supplying a feed stream including a propylene monomer and water to a reaction unit and allowing a reaction to proceed to produce a reaction product including isopropyl alcohol, a propylene monomer, and water;
supplying each of a first stream including a vapor phase reaction product and a second stream including a liquid phase reaction product that are discharged from the reaction unit to an absorption column of a purification unit; and
circulating a third stream of vapor phase including the propylene monomer discharged through an upper portion of the absorption column to the reaction unit, and supplying a fourth stream of liquid phase including water and isopropyl alcohol discharged through a lower portion of the absorption column to an isopropyl alcohol purification unit,
wherein the purification unit includes one or more heat exchangers, and
when the third stream is circulated to the reaction unit, the third stream is converted from a vapor phase to a liquid phase through the one or more heat exchangers provided in the purification unit, and then transferred.

2. The method of claim 1, wherein when the first stream is fed to the absorption column,
the first stream is partially condensed through a first heat exchanger provided in the purification unit, and the partially condensed first stream is fed to the absorption column as a vapor-liquid mixed phase.

3. The method of claim 2, wherein the third stream converted to the liquid phase is heat-exchanged with the first stream through the first heat exchanger.

4. The method of claim 1, wherein the liquid phase conversion of the third stream includes:
primarily condensing the third stream discharged through the upper portion of the absorption column through a second heat exchanger provided in the purification unit; and
secondarily condensing the primarily condensed third stream through a third heat exchanger provided in the purification unit.

5. The method of claim 4, wherein the primarily condensed third stream is a vapor-liquid mixed phase stream, and
the secondarily condensed third stream is a liquid phase stream.

6. The method of claim 4, wherein a temperature of the primarily condensed third stream is 90°C to 100°C.

7. The method of claim 4, wherein a temperature of the secondarily condensed third stream is 50°C to 70°C.

8. The method of claim 4, wherein the third stream discharged through the upper portion of the absorption column and the secondarily condensed third stream are heat-exchanged with each other through the third heat exchanger provided in the purification unit.

9. The method of claim 8, wherein the secondarily condensed third stream, after passing through the third heat exchanger, is a vapor-liquid mixed phase stream.

10. The method of claim 1, wherein the third stream converted to the liquid phase is fed to the reaction unit using a pump.

11. The method of claim 1, wherein the reaction unit includes a reactor and one or more heat exchangers,
the feed stream is fed and a reaction is allowed to proceed in the reactor to form a vapor phase reaction product, and
a vapor phase reaction product stream discharged from the reactor passes through the one or more heat exchangers provided in the reaction unit and is separated into a first stream including a vapor phase reaction product and a second stream including a liquid phase reaction product, and the first stream and the second stream are each fed to the absorption column.

12. The method of claim 11, wherein the feed stream passes through the one or more heat exchangers before being fed to the reactor, and
a temperature of the feed stream, after passing through the one or more heat exchangers, is 160°C to 180°C.

13. The method of claim 1, wherein a temperature of each of the first stream and the second stream is 110°C to 130°C.
